# EUROPÄISCHE PATENTSCHRIFT

(11) **EP 2 565 013 B1**
(45) Veröffentlichungstag und Bekanntmachung des Hinweises auf die Patenterteilung: **26.11.2014**
(21) Anmeldenummer: 11179885.6
(22) Anmeldetag: 02.09.2011
(51) Int. Cl.: B29C 55/06, A61F 13/15, B32B 37/20

(54) **Verfahren zum Recken einer Folienbahn**
Method for stretching a sheet of film
Procédé de tension d'une bande de feuille

(43) Veröffentlichungstag der Anmeldung: 06.03.2013
(60) Teilanmeldung: 13198933.7
(73) Patentinhaber: RKW SE, 67227 Frankenthal (DE)
(72) Erfinder: Börmann, Ludwig, 83547 Babensham (DE); Schreiner, Günter, 83530 Schnaitsee (DE)
(74) Vertreter: Bublak, Wolfgang

(56) Entgegenhaltungen:
- EP-A1- 0 951 080
- EP-A1- 1 716 830
- WO-A1-01/09424
- WO-A1-03/008190
- WO-A1-2010/112418
- WO-A1-2010/128124
- WO-A2-2004/094129

## Beschreibung

Die Erfindung betrifft ein Verfahren zur Herstellung von Vlies-Folien-Laminaten, damit hergestellte Vlies-Folien-Laminate sowie ihre Verwendung, beispielsweise im Hygienesektor.

Im Zuge der Umweltdiskussion zur Ressourcenschonung und Nachhaltigkeit wird es auf dem Gebiet der Folien, vor allem von Folien für Wegwerfprodukte aus dem Hygienesektor, von immer größerer Bedeutung, noch dünnere Folien als bisher herzustellen, um Rohstoffe einzusparen.

Aus der EP-A-0 768 168 und der EP-A-1 716 830 sind Verfahren zur Herstellung von im Hygienebereich einsetzbaren Folien bekannt. An solche Hygienefolien werden aufgrund ihres Einsatzbereichs mehrere Anforderungen gestellt. Sie sollen flüssigkeitsdicht sein und über bestimmte haptische Eigenschaften verfügen, wie Weichheit, Geschmeidigkeit, raschelarmes Verhalten und textiler Griff. Folien im Hygienebereich sollen einen weichen, stoffähnlichen Griff besitzen. Insbesondere bei ihrer Verwendung für Inkontinenzprodukte soll die Geräuschentwicklung möglichst gering sein, d.h. die Folien sollen raschelarm sein. In Verbindung mit einem geringen Glanzgrad ergibt dies eine sehr textile Folie, was im Hygienebereich erwünscht ist. Hinzu kommt, dass in den letzten Jahren die in Windeln und Inkontinenzprodukten enthaltenen Saugkörper immer dünner geworden sind, was insbesondere durch die Verwendung von Superabsorber-Polymeren ermöglicht wurde. Diese Superabsorber-Polymere werden in Form grobkörniger Pulver eingesetzt, und die Hygienefolien müssen eine derartige Festigkeit besitzen, dass ein Durchstoßen der Folie durch die einzelnen Körner, z.B. bei Belastung durch Hinsetzen oder sonstige Bewegung des Trägers, mit Sicherheit vermieden wird. Eine Bildung von Löchern ("*Pinholes*") durch Superabsorber-Polymere und ein Platzen der Fertigfolienprodukte in den Verpackungseinheiten muss vermieden werden. Eine weitere Anforderung an Hygienefolien besteht in einer Mindestzugfestigkeit, die für eine Verarbeitung der Folienbahnen auf den schnell laufenden Maschinen (Konverter) der Hersteller von z.B. Windeln und Damenbinden erforderlich ist. Diese Mindestzugfestigkeit wird für 5%, 10 % oder 25 % Dehnung in Maschinenrichtung (*md*) oder Querrichtung (*cd*) angegeben. Derzeit soll die Zugfestigkeit bei 5 % Dehnung (5%-Modul) in Maschinenrichtung wenigstens 2,5 N/inch betragen. Darüber hinaus sollen Folien für Hygieneanwendungen eine Längs- und Querreißfestigkeit von wenigstens 10 N/inch aufweisen.

Bekannt ist auch die Verwendung von Laminaten aus Folie und Vlies. Eine Herstellung derartiger Laminate ist in der WO 2006/024394 beschrieben, bei der eine Ausgangsfolienbahn aus thermoplastischem Polymermaterial zusammen mit einer Ausgangsvliesbahn, deren Schmelzpunkt oberhalb des Kristallitschmelzpunktes des Polymermaterials liegt, auf eine Temperatur über dem Kristallitschmelzpunkt des Polymermaterials und unter dem Schmelzpunkt der Ausgangsvliesbahn erwärmt und das gebildete Laminat durch einen gekühlten Walzenspalt geführt und dabei auf eine Temperatur unterhalb des Kristallitschmelzpunktes der Ausgangfolienbahn gekühlt wird.

In der EP-A-0 768 168 wird eine Ausgangsfolienbahn aus thermoplastischem Polymermaterial bis zum schmelzeflüssigen Zustand des Polymermaterials erwärmt und danach durch einen gekühlten Walzenspalt geführt. In der EP-A-1 716 830 wird ein Verfahren mit Erwärmen des Polymermaterials und anschlie-βendes Führen durch einen gekühlten Walzenspalt mit einer Ausgangsfolienbahn durchgeführt, die ein thermoplastisches Polymermaterial mit einer Polyethylen-Matrix enthält, in der 1 bis 70 Gewichtsteile Polypropylen, bezogen auf 100 Gewichtsteile Polyethylen-Matrix enthalten sind. Hierbei wird die Erwärmung der Ausgangsfolienbahn bis zum schmelzeflüssigen Zustand des Polyethylen-Matrixmaterials, jedoch nicht bis zum schmelzeflüssigen Zustand des Polypropylens durchgeführt. Darin beschrieben sind Folien mit geringen Dicken bis zu 15 µm herab, die noch die Anforderungen an Hygienefolien erfüllen.

Um die Dicke von Folien zu verringern, ist aus dem Stand der Technik das Recken oder Verstrecken von Folienbahnen bekannt. So ist aus der Auslegeschrift DE 1 108 420 ein Verfahren zur Herstellung von thermoplastischen Folien bekannt, bei dem eine allseitige Verstreckung der Folie durchgeführt wird, wobei die Verstreckung in einem Temperaturbereich vom Kristallitschmelzpunkt oder Erweichungspunkt bis 60°C unterhalb dieser Punkte vorgenommen wird. Die Offenlegungsschrift DE 1 704 538 betrifft ein Verfahren zum einachsigen Recken von Polypropylenfilmen, bei dem das Recken in zwei oder mehreren aufeinanderfolgenden Stufen unter Einhaltung bestimmter Reaktionsbedingungen bis zu einem endgültigen Ziehverhältnis von 6:1 durchgeführt wird. Die Offenlegungsschrift DE 2 257 089 beschreibt ein Verfahren zum einachsigen Recken von thermoplastischen Folien, bei dem eine Verringerung der Bahnbreiten vermieden und eine gleichmäßige Folie erhalten wird, indem der Folie in definierter Weise Erweichungswärme beim Reckvorgang zugeführt wird. Die DE 600 08 145 T2 ist auf ein Verfahren zum Strecken einer Folie, beispielsweise einer thermoplastischen Folie gerichtet, bei dem eine Apparatur mit angetriebenen und nicht angetrieben Haltern eingesetzt wird. Daraus sich ergebende Ungleichmäßigkeiten der Halterabstände sollen darin durch eine bestimmte Kühlung minimiert werden.

Die Aufgabe der Erfindung bestand nun darin, noch dünnere Folien herzustellen, als sie beispielsweise gemäß der EP-A-1 716 830 erhältlich sind, um Rohstoffe einzusparen, und welche zu Hygieneprodukten weiterverarbeitet werden können.

Erfindungsgemäß wurde gefunden, dass Folien aus thermoplastischem Polymermaterial, die eine niedrig schmelzende Komponente und eine hoch schmelzende Komponente enthalten, überraschenderweise stark gereckt werden können, wenn sie durch Erwärmung in den teilweise geschmolzenen Zustand übergeführt werden, so dass sich die niedrig schmelzende Komponente, jedoch nicht die hoch schmelzende Komponente im schmelzeflüssigen Zustand befindet, und anschließend in einem gekühlten Walzenspalt abgekühlt werden. Das Recken wird dabei zwischen einer für die Erwärmung eingesetzten Heizwalze und dem Kühlwalzenspalt bewirkt. Auf diese Weise kann eine signifikante Verringerung der Foliendicke erreicht werden. Dies ermöglicht eine Prozess-stabile Herstellung von ultradünnen Folien mit Dicken von bis zu 5 µm bzw. 4 g/m² oder sogar darunter und führt zu wirtschaftlich interessanten Rohstoffeinsparungen. Es war überraschend, dass durch Erwärmen der Folienbahn bis zu deren teilweise schmelzeflüssigem Zustand ein derartig starkes Recken möglich ist.

Somit betrifft die Erfindung ein Verfahren zum Recken einer Ausgangsfolienbahn aus thermoplastischem Polymermaterial, das wenigstens eine niedrig schmelzende Polymerkomponente und wenigstens eine hoch schmelzende Polymerkomponente enthält, wobei das Verfahren folgende Schritte umfasst: Erwärmen der Ausgangsfolienbahn bis zum teilweise geschmolzenen Zustand, bei dem sich die wenigstens eine niedrig schmelzende Polymerkomponente im schmelzeflüssigen Zustand und die wenigstens eine hoch schmelzende Polymerkomponente nicht im schmelzeflüssigen Zustand befindet, durch wenigstens eine Heizwalze, und Abkühlen durch Führen der teilweise geschmolzenen Folienbahn durch einen gekühlten Walzenspalt, wobei die Folienbahn zwischen der wenigstens einen Heizwalze und dem gekühlten Walzenspalt gereckt wird.

In einer bevorzugten Ausführungsform der Erfindung werden die den gekühlten Walzenspalt bildenden Kühlwalzen bei einer höheren Geschwindigkeit als die wenigstens eine Heizwalze angetrieben.

In einer anderen bevorzugten Ausführungsform der Erfindung sind vor dem Kühlwalzenspalt zwei Walzen vorhanden, die mit unterschiedlicher Geschwindigkeit betrieben werden, so dass die Folienbahn zwischen der ersten und zweiten Walze gereckt wird, und wobei wenigstens die erste der zwei Walzen als Heizwalze ausgebildet ist. Bei einer bevorzugten Ausgestaltung dieser Ausführungsform kann, wenn neben der ersten Walze auch die zweite Walze als Heizwalze ausgebildet ist, zusätzlich eine Vliesbahn über diese zweite Heizwalze mitgeführt und zusammen mit der Folienbahn durch den gekühlten Walzenspalt geführt werden, so dass ein Vlies-Folien-Laminat erhalten wird.

Außerdem betrifft die Erfindung ein Vlies-Folien-Laminat, das durch Verkleben einer erfindungsgemäß erhaltenen Folie mit einem Vlies hergestellt wird.

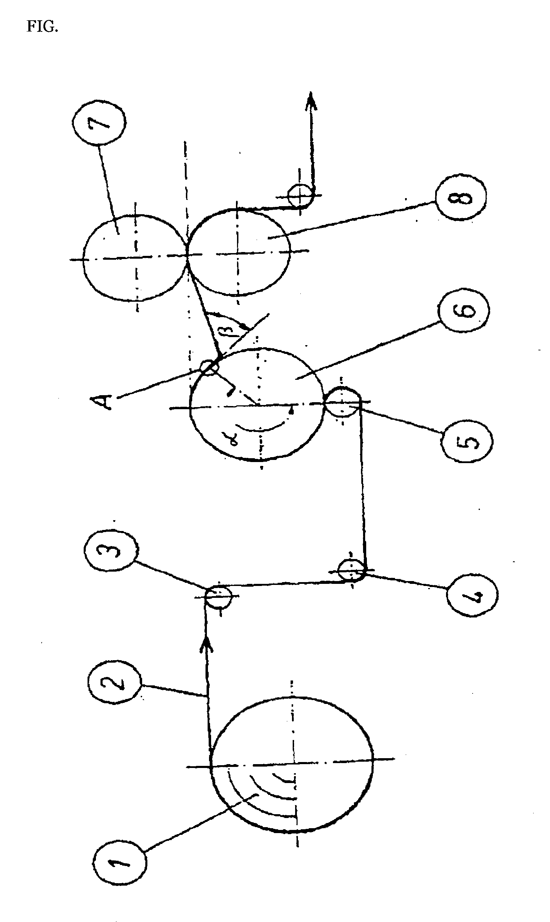

Weiterhin betrifft die Erfindung die mit den beschriebenen Verfahren hergestellten Folienbahnen und Laminate sowie deren Verwendung, insbesondere im Hygiene- oder Medikalbereich. Bevorzugte Ausführungsformen der Erfindung sind in der nachfolgenden Beschreibung, der Figur, dem Beispiel und den Unteransprüchen beschrieben.

Das Verfahren der Erfindung ermöglicht die Herstellung von äußerst dünnen, kommerziell und technisch verwertbaren Folien. Beispielsweise können Folien mit einer Dicke von unter 10 µm, z. B. 8 µm oder 6 µm oder 5 µm oder sogar darunter, beispielsweise 2 µm, in einem stabilen Prozess hergestellt werden. Solche Folien können z.B. inline zu Laminaten als sogenannte Backsheets mit textilem Griff (*Textile Backsheets*) für Windeln weiterverarbeitet werden. Ein weiterer Vorteil der nach dem Verfahren der Erfindung erhaltenen Folienbahnen besteht aufgrund des Einsatzes der hoch schmelzenden Polymerkomponente, z.B. von Polypropylen, in einer verbesserten Thermostabilität. Dies ermöglicht beispielsweise bei der Verwendung der Folienbahnen als Backsheets im Hygienebereich, dass die Innenausstattung von z. B. Babywindeln oder Inkontinenzartikeln mittels Hotmelt-Klebersystemen bei Temperaturen im Bereich von 140 bis 160 °C aufgebracht werden kann, ohne dass dadurch das dünne Folien-Backsheet angeschmolzen wird.

In der vorliegenden Erfindung beziehen sich die angegebenen Schmelzpunkte, Schmelzbereiche und Kristallitschmelzpunkte auf eine Bestimmung gemäß DSC (Differential Scanning Calorimetry).

Erfindungsgemäß enthält oder umfasst die Ausgangsfolienbahn wenigstens eine niedrig schmelzende Polymerkomponente und wenigstens eine hoch schmelzende Polymerkomponente. Mit anderen Worten, die Ausgangsfolienbahn enthält eine oder mehrere niedrig schmelzende Polymerkomponente(n) und eine oder mehrere hoch schmelzende Polymerkomponente(n). Das Gleiche bedeuten die in der vorliegenden Erfindung weiterhin verwendeten Begriffe "eine niedrig schmelzende Polymerkomponente" und "eine hoch schmelzende Polymerkomponente", d.h. auch sie umfassen eine oder mehrere niedrig bzw. hoch schmelzende(n) Polymerkomponente(n). Vorzugsweise enthält die Ausgangsfolienbahn eine, bevorzugt zwei, niedrig schmelzende Polymerkomponente(n). Bevorzugt enthält sie eine, insbesondere zwei, hoch schmelzende Polymerkomponente(n). In anderen Ausführungsformen der Erfindung enthält sie bevorzugt drei niedrig schmelzende Polymerkomponenten und/oder drei hoch schmelzende Polymerkomponenten. Ob ein Polymermaterial der Ausgangsfolienbahn zur niedrig schmelzenden Polymerkomponente oder zur hoch schmelzenden Polymerkomponente gehört, bestimmt sich erfindungsgemäß nach dem jeweiligen Kristallitschmelzpunkt, Schmelzpunkt oder Schmelzbereich des Polymermaterials im Verhältnis zur Recktemperatur. Bei gegebener Recktemperatur werden die schmelzeflüssigen Polymermaterialien der niedrig schmelzenden Polymerkomponente und die nicht-schmelzeflüssigen Polymermaterialien der hoch schmelzenden Polymerkomponente zugeordnet.

Bekanntlich besitzen Polymere keinen scharf definierten Schmelzpunkt, sondem einen Schmelzbereich, wobei sich jedoch den kristallinen Bereichen eines Polymeren ein Kristallitschmelzpunkt zuordnen lässt. Dieser Kristallitschmelzpunkt liegt stets höher als der Schmelzpunkt oder Schmelzbereich der nichtkristallinen Bestandteile. Der schmelzeflüssige Zustand ist dadurch beschrieben, dass der Schubmodul gegen Null geht. Im Falle von Polymeren mit kristallinen Bereichen sind letztere dann nicht mehr nachweisbar. Der Schubmodul kann beispielsweise nach ISO 6721-1 & 2 bestimmt werden. Bei der vorliegenden Erfindung wird die Ausgangsfolienbahn auf eine Temperatur erwärmt, bei der für die niedrig schmelzende Polymerkomponente der Schubmodul Null beträgt und für die hoch schmelzende Polymerkomponente der Schubmodul nicht Null ist. Bei der niedrig schmelzenden Polymerkomponente sind dann keine kristallinen Bereiche mehr nachweisbar und die niedrig schmelzende Polymerkomponente befindet sich im schmelzeflüssigen Zustand. Hingegen sind bei der hoch schmelzenden Polymerkomponente noch kristalline Bereiche nachweisbar und diese befindet sich unterhalb des schmelzeflüssigen Zustands. In Summe ist der Schubmodul des gesamten Polymermaterials der Ausgangsfolienbahn somit nicht Null und kristalline Bereiche der hoch schmelzenden Polymerkomponente sind noch nachweisbar. Es liegt also eine teilweise aufgeschmolzene Folienbahn vor.

Als Materialien für die beiden Polymerkomponenten der Ausgangsfolienbahn kommen im Prinzip alle thermoplastischen Polymere in Betracht, die über entsprechende Schmelzpunkte verfügen. Hierzu sind zahlreiche Handelsprodukte auf dem Markt erhältlich. Vorzugsweise werden verschiedene Polyolefine, insbesondere Polyethylene, Polypropylene, Copolymerisate von Ethylen und Propylen, Copolymerisate von Ethylen und Propylen mit anderen Comonomeren, oder Gemische davon eingesetzt. Weiterhin sind Ethylenvinylacetat (EVA), Ethylenacrylat (EA), Ethylenethylacrylat (EEA), Ethylenacrylsäure (EAA), Ethylenmethylacrylat (EMA), Ethylenbutylacrylat (EBA), Polyester (PET), Polyamid (PA), z.B. Nylon, Ethylenvinylalkohole (EVOH), Polystyrol (PS), Polyurethan (PU) oder thermoplastische Olefinelastomere geeignet.

Die Gesamtmenge an niedrig schmelzender Polymerkomponente beträgt vorzugsweise 90 bis 30 Gew.-%, insbesondere 80 bis 40 Gew.-%, am meisten bevorzugt 70 bis 50 Gew.-%, und die Gesamtmenge an hoch schmelzender Polymerkomponente beträgt vorzugsweise 10 bis 70 Gew.-%, insbesondere 20 bis 60 Gew.-%, am meisten bevorzugt 30 bis 50 Gew.-%, jeweils bezogen auf 100 Gew.-% niedrig schmelzende und hoch schmelzende Polymerkomponente. Altemativ beträgt vorzugsweise die Gesamtmenge an niedrig schmelzender Polymerkomponente 85 bis 15 Gew.-% und die Gesamtmenge an hoch schmelzender Polymerkomponente 15 bis 85 Gew.-%, wiederum bezogen auf 100 Gew.-% niedrig und hoch schmelzende Komponente. Bei diesen Mengenangaben kann es sich beispielsweise bei der niedrig schmelzenden Polymerkomponente um ein oder mehrere Polyethylen(e) und bei der hoch schmelzenden Polymerkomponente um ein oder mehrere Polypropylen(e) handeln.

In einer besonders bevorzugten Ausführungsform enthält die Ausgangsfolienbahn wenigstens ein Polyethylen als niedrig schmelzende Polymerkomponente und wenigstens ein Polypropylen als hoch schmelzende Polymerkomponente.

Vorzugsweise enthält die niedrig schmelzende Polymerkomponente Ethylenpolymere oder besteht aus diesen, wobei sowohl Ethylenhomopolymere als auch Ethylencopolymere mit Ethylen als Hauptmonomerem sowie Gemische (Blends) von Ethylenhomopolymeren und Ethylencopolymeren geeignet sind. Geeignete Ethylenhomopolymere sind LDPE (Low Density Polyethylene), LLDPE (Linear Low Density Polyethylene), MDPE (Medium Density Polyethylene) und HDPE (High Density Polyethylene). Bevorzugte Comonomere für Ethylencopolymere sind andere Olefine als Ethylen mit Ausnahme von Propylen, z. B. Buten, Hexen oder Octen. Vorzugsweise liegt bei den Ethylencopolymeren der Comonomergehalt unter 20 Gew.-%, insbesondere unter 15 Gew.-%. In einer bevorzugten Ausführungsform besteht die niedrig schmelzende Polymerkomponente ausschließlich aus Ethylenhomopolymeren, z. B. Gemischen aus LDPE und LLDPE, die jeweils in Mengen von 10 bis 90 Gew.-% enthalten sein können, sowie 0 bis 50 Gew.-% MDPE. Spezielle Beispiele sind ein Polyethylen aus 60 Gew.-% LDPE und 40 Gew.-% LLDPE oder ein Polyethylen aus 80 Gew.-% LDPE und 20 Gew.-% LLDPE.

Neben den Ethylenhomopolymeren und/oder Ethylencopolymeren kann die niedrig schmelzende Polymerkomponente auch andere thermoplastische Polymere enthalten. Diese thermoplastischen Polymeren sind nicht beschränkt, solange hierdurch die Temperatur, bei der sich die gesamte niedrig schmelzende Polymerkomponente im schmelzeflüssigen Zustand befindet, nicht zu nahe an die Temperatur heranrückt, bei der sich die hoch schmelzende Polymerkomponente im schmelzeflüssigen Zustand befindet. Es ist auch möglich, dass die niedrig schmelzende Polymerkomponente ein Polypropylen enthält, dessen Schmelzpunkt oder Schmelzbereich nicht höher als der eines Ethylenhomopolymeren oder Ethylencopolymeren liegt oder aber zwar höher als diese, jedoch immer noch tiefer als die eingesetzte Recktemperatur. Bekanntlich gibt es hochkristallines isotaktisches, weniger kristallines syndiotaktisches und amorphes ataktisches Polypropylen, die über unterschiedliche Schmelzpunkte, Schmelzbereiche oder Kristallischmelzpunkte verfügen. Bei Verwendung von amorphem ataktischem Polypropylen, das einen wesentlich niedrigeren Schmelzpunkt oder Schmelzbereich als isotaktisches und gegebenenfalls auch syndiotaktisches Polypropylen hat, wäre dieses in Abhängigkeit von der Recktemperatur gegebenenfalls der niedrig schmelzenden Polymerkomponente zuzurechnen.

Vorzugsweise enthält die hoch schmelzende Polymerkomponente wenigstens ein Polypropylen, dessen Schmelzpunkt, Schmelzbereich oder Kristallitschmelzpunkt wesentlich höher als der der niedrig schmelzenden Polymerkomponente liegt. Geeignetes Polypropylen ist insbesondere isotaktisches Polypropylen. Es kann auch syndiotaktisches Polypropylen verwendet werden, sofern dessen Schmelzpunkt, Schmelzbereich oder Kristallitschmelzpunkt wesentlich höher als der der niedrig schmelzenden Polymerkomponente liegt. Geeignete Polypropylene sind im Handel erhältlich, beispielsweise für die Herstellung von Blas- und/oder Cast-Folien.

Die hoch schmelzende Polymerkomponente kann sowohl Propylenhomopolymere als auch Propylencopolymere mit Propylen als Hauptmonomerem umfassen. Bei den Propylencopolymeren ist hierbei der Anteil des Comonomeren, d. h. das Nicht-Propylen, in Anhängigkeit von den anderen Komponenten und der Recktemperatur der niedrig oder hoch schmelzenden Polymerkomponente zuzurechnen. Geeignete Comonomere für Propylencopolymere sind andere Olefine als Propylen, vorzugsweise Ethylen. Bei den Propylen-Ethylen-Copolymeren beträgt der Ethylenanteil vorzugsweise 2 bis 30 Gew.-%, besonders bevorzugt 2 bis 20 Gew.-% und insbesondere 2 bis 15 Gew.-%, wobei in der Praxis bei einem Ethylengehalt von 3 bis 20 Gew.-% sehr gute Ergebnisse erhalten werden. Diese Zahlenwerte gelten auch für die anderen Olefine.

Nachfolgend sind für einige Polyethylene und Polypropylene die Schmelzbereiche angegeben:
LDPE: 110 - 114°C;
LLDPE: 115 - 130°C;
HDPE: 125 - 135 °C;
Propylen-Homopolymere: 150 - 165°C;
Propylen-Ethylen-Copolymere: 120 - 162°C, bei sehr wenig Ethylen auch höhere Temperaturen möglich;
Bimodale Propylen-Ethylen-(Homo)Copolymere: 110 - 165 °C.

Es ist auch möglich, sogenannte bimodale Polypropylene zu verwenden. Dabei handelt es sich um zwei verschiedene Polypropylene mit jeweils unterschiedlichem Copolymeranteil, die in einem Rohstoff zusammengefasst sind. Ein solches bimodales Polypropylen hat zwei Kristallitschmelzpunkte, wobei in der Regel über eine DSC-Analyse auch die ungefähren Anteile der zwei Polypropylene bestimmt werden können. Als Beispiel sei ein bimodales Polypropylen mit den Kristallitschmelzpunkten 125 °C und 143 °C mit einem Anteil der zwei verschiedenen Polypropylene von 25/75 genannt. Bei einer Recktemperatur von 130 °C wären erfindungsgemäß die 25 % Polypropylen mit Kristallitschmelzpunkt 125 °C der niedrig schmelzenden Polymerkomponente und die 75 % Polypropylen mit Kristallitschmelzpunkt 143 °C der hoch schmelzenden Polymerkomponente zuzurechnen.

Bei dem Verfahren der Erfindung wird die Erwärmung der Ausgangsfolienbahn bis zum oder oberhalb dem schmelzeflüssigen Zustand der niedrig schmelzenden Polymerkomponente und unterhalb des schmelzeflüssigen Zustands der hoch schmelzenden Polymerkomponente durchgeführt. Bis zum schmelzeflüssigen Zustand bedeutet hierbei, dass sich die niedrig schmelzende Polymerkomponente im schmelzeflüssigen Zustand befindet. Es wird jedoch nur so hoch erwärmt, dass sich die hoch schmelzende Polymerkomponente nicht im schmelzeflüssigen Zustand befindet.

In einer besonderen Ausführungsform wird eine Ausgangsfolienbahn folgender Zusammensetzung verwendet: 25 bis 35 Gew.-% eines Ethylen-Octen-Copolymeren mit 5 bis 15 Gew.-% Octenanteil; 20 bis 30 Gew.-% eines Propylen-Ethylen-Copolymeren mit 3 bis 12 Gew.-% Ethylen, und Rest LDPE, bezogen auf 100 Gew.-% niedrig und hoch schmelzende Polymerkomponente.

Ebenso wie sich in der niedrig schmelzenden Polymerkomponente spezielles geschmolzenes Polypropylen befinden kann, kann sich auch in der hoch schmelzenden Polymerkomponente ein spezielles nicht geschmolzenes Polyethylen befinden, das dann der hoch schmelzenden Polymerkomponente zugerechnet wird. Dies wird an folgendem Beispiel veranschaulicht. Eine für eine Ausgangsfolienbahn geeignete Rezeptur umfasst 30 Gew.-% LDPE (Schmelzpunkt 112 °C), 30 Gew.-% LLDPE (Schmelzpunkt 124 °C), 20 Gew.-% HDPE (Schmelzpunkt 130 °C) und 20 Gew.-% Polypropylen (Schmelzpunkt 160 °C). Wird die Folienbahn bei einer Temperatur von 126 °C gereckt, so befinden sich das LDPE und LLDPE erfindungsgemäß im schmelzeflüssigen Zustand, aber nicht nur das Polypropylen sondern auch das HDPE befinden sich nicht im schmelzeflüssigen Zustand.

Um eine stabile Prozessführung auch über einen längeren Zeitraum zu ermöglichen, sollten zweckmäßigerweise die (Kristallit-)Schmelzpunkte der niedrig und hoch schmelzenden Polymerkomponenten nicht zu nahe beieinander liegen. Vorzugsweise liegt der Kristallitschmelzpunkt der niedrig schmelzenden Polymerkomponente oder, bei Anwesenheit mehrerer niedrig schmelzender Polymerkomponenten, der Kristallitschmelzpunkt derjenigen mit dem höchsten Kristallitschmelzpunkt davon, mindestens etwa 5 °C, verzugsweise mindestens etwa 10 °C und insbesondere mindestens etwa 20 °C unterhalb des Kristallitschmelzpunkts oder des schmelzeflüssigen Zustandes der hoch schmelzenden Polymerkomponente oder, bei Anwesenheit mehrerer hoch schmelzender Polymerkomponenten, derjenigen mit dem niedrigsten Kristallitschmelzpunkt davon.

Das erfindungsgemäße Verfahren ermöglicht auch die Herstellung atmungsaktiver Folien. In diesem Fall enthalten die Folien zusätzlich Füllstoffe, an denen sich beim Reckvorgang Poren ausbilden können. Geeignete Füllstoffe sind dem Fachmann bekannt. Am meisten bevorzugt ist Calciumcarbonat oder Kreide wegen seines günstigen Preises, aber auch unter dem Gesichtspunkt der Nachhaltigkeit. Falls ein Füllstoff mit einer gleichmäßigeren Partikelgröße als bei Kreide gewünscht ist, besteht auch die Möglichkeit, synthetische Füllstoffe mit einheitlicher Partikelgröße oder Partikelgrößenverteilung zu verwenden. Um eine Atmungsaktivität der Folie zu erreichen, werden zweckmäßigerweise wenigstens 40 Gew.-% Füllstoff, insbesondere wenigstens 50 Gew.-% Füllstoff, bezogen auf die gesamte Rezeptur der Ausgangsfolienbahn (100 Gew.-%, einschließlich Füllstoff) verwendet. Die Obergrenze an Füllstoff wird dadurch bestimmt, dass keine Poren mehr, sondern Löcher entstehen, oder die Folie reißt. Geeignete Folienrezepturen mit Füllstoff können vom Fachmann routinemäßig bestimmt werden. Besonders geeignet ist eine Rezeptur mit 40 bis 75 Gew.-%, insbesondere 50 bis 75 Gew.-%, Füllstoff bezogen auf 100 Gew.-% Ausgangsfolienbahn. Dabei ist darauf zu achten, den Anteil der niedrig schmelzenden Komponente nicht so hoch zu wählen, dass eine Atmungsaktivität nicht erreicht wird oder aber wieder verloren geht, weil die Poren nicht erzeugt werden bzw. sich wieder verschließen. Daneben besteht auch die Möglichkeit, Füllstoffe in einer geringeren Menge einzusetzen als sie für eine Atmungsaktivität der Folie erforderlich sind. Solche Folien können unter dem Gesichtspunkt der Nachhaltigkeit von Interesse sein. Geeignete Rezepturen sind 1 bis 75 Gew.-%, insbesondere 10 bis 75 Gew.-%, Füllstoff bezogen auf 100 Gew.-% Ausgangsfolienbahn.

Zur Erreichung des schmelzeflüssigen Zustands der niedrig schmelzenden Polymerkomponente, jedoch nicht des schmelzeflüssigen Zustands der hoch schmelzenden Polymerkomponente, unterliegt der speziell gewählte Temperaturabstand keinen besonderen Beschränkungen, sofern die vorgenannte Bedingung erfüllt ist. Der gewählte Temperaturabstand wird zweckmäßigerweise durch praktische Überlegungen bezüglich Sicherheit der Verfahrensdurchführung, z.B. auch beim Recken, oder durch wirtschaftliche Überlegungen bestimmt. Wenn z.B. bei einer bestimmten Temperatur die niedrig schmelzende Polymerkomponente aufgeschmolzen ist, bringt eine weitere Temperaturerhöhung keine besseren Ergebnisse. Zudem steigt der Wärmeverbrauch und man kommt gegebenenfalls zu nahe an den Schmelzbereich der hoch schmelzenden Polymerkomponente heran, so dass das Verfahren schwieriger durchzuführen ist. Vorzugsweise wird deshalb das Verfahren der Erfindung so durchgeführt, dass die Erwärmung der Ausgangsfolienbahn auf 5 bis 20 °C, bevorzugt 5 bis 15 °C oder 10 bis 20°C, insbesondere 10 bis 15°C oder 15 bis 20 °C, unterhalb des Kristallitschmelzpunkts der hoch schmelzenden Polymerkomponente erfolgt. Alternativ wird die Erwärmung insbesondere bei einer Temperatur im Bereich von 1 bis 20 °C, bevorzugt 2 bis 10°C, oberhalb des Kristallitschmelzpunkts oder schmelzeflüssigen Zustands der niedrig schmelzenden Polymerkomponente(n) durchgeführt. Es ist sicherzustellen, dass die Kristallitschmelzpunkte der niedrig schmelzenden Polymerkomponente(n) erreicht werden.

Die Ausgangsfolienbahnen für die Durchführung des Verfahrens der Erfindung können durch beliebige, im Stand der Technik bekannte Verfahren hergestellt werden. Beispielsweise kann die Ausgangsfolienbahn durch Verschmelzen der Polymerbestandteile im Extruder auf eine Temperatur deutlich über der Schmelzflusstemperatur aller Bestandteile (z.B. über 200°C) und anschließendes Schlitzdüsenverfahren oder Blasverfahren hergestellt werden. Beim Schlitzdüsenverfahren wird eine Folie durch eine Breitschlitzdüse extrudiert. Bevorzugt ist das Blasverfahren.

Die Ausgangsfolienbahn kann einschichtig oder mehrschichtig sein, also mono- bzw. coextrudiert, wobei es keine Beschränkung bezüglich der Anzahl der verwendeten Schichten gibt. Die Schichten können gleiche oder unterschiedliche Rezepturen aufweisen, wobei die Zuordnung zur niedrig oder hoch schmelzenden Polymerkomponente jeweils durch den Kristallitschmelzpunkt im Verhältnis zur Recktemperatur bestimmt wird.

Die beim Verfahren der Erfindung eingesetzten Ausgangsfolienbahnen können eingefärbt sein, z. B. weiß mit Titandioxid. Weiterhin können die Ausgangsfolienbahnen übliche Zusatzstoffe und Verarbeitungshilfsmittel enthalten. Insbesondere handelt es sich hierbei neben den bereits genannten Füllstoffen um Pigmente oder andere farbgebende Stoffe, Antihaftmittel, Gleitmittel, Weichmacher, Verarbeitungshilfsmittel, antistatische Mittel, keimverhindernde Mittel (Biozide), Antioxidationsmittel, Wärmestabilisierungsmittel, Stabilisierungsmittel gegenüber UV-Licht oder andere Mittel zur Eigenschaftsmodifizierung. Typischerweise werden solche Stoffe, ebenso wie Füllstoffe, bereits vor dem erfindungsgemäßen Recken der Ausgangsfolienbahn zugegeben, z.B. bei deren Herstellung in die Polymerschmelze oder vor dem Extrudieren zu einer Folie.

Die Dicke der Ausgangsfolienbahn liegt bei nicht gefüllten Folien insbesondere im Bereich von unter 30 µm, vorzugsweise unter 20 µm, am stärksten bevorzugt unter 15 µm. Bevorzugt sind Bereiche von 10 bis 20 µm und besonders bevorzugt 10 bis 15 µm. Weiterhin bevorzugt liegt der Bereich bei 10 bis 30 µm, was in Abhängigkeit von der Dichte Flächengewichten von 9 bis 29 g/m² entspricht. Bei atmungsaktiven Ausgangsfolien (gefüllte Folien) liegen bevorzugte Flächengewichte im Bereich unter 50 g/m², insbesondere unter 40 g/m², besonders bevorzugt unter 30 g/m² und stärker bevorzugt unter 20 g/m².

Erfindungsgemäß erfolgt die Erwärmung der Ausgangsfolienbahn mittels wenigstens einer Heizwalze. Vorzugsweise erfolgt die Erwärmung mittels einer oder mehrerer Heizwalzen, bei denen es sich um Kontaktwalzen handelt, die mittels eines Wärmeträgers, z.B. Dampf, Wasser, Öl, auf die vorgegebene Temperatur erhitzt werden. In einer bevorzugten Ausführungsform wird eine einzelne Heiz- oder Kontaktwalze eingesetzt. Es ist aber auch möglich, zwei oder mehrere Heizwalzen einzusetzen, wobei sichergestellt werden muss, dass der schmelzeflüssige Zustand der niedrig schmelzenden Polymerkomponente vor dem Kühlwalzenspalt erreicht wird. Um zu gewährleisten, dass die Ausgangsfolienbahn die Temperatur der Heizwalze tatsächlich erreicht oder dass bei hohen Produktionsgeschwindigkeiten (bei denen die Oberflächentemperatur des Heizzylinders höher im Vergleich zur Folie ist) der schmelzeflüssige Zustand der niedrig schmelzenden Polymerkomponente sicher erreicht wird, ist für eine ausreichende Verweilzeit der Ausgangsfolienbahn auf der Heizwalzenoberfläche zu sorgen. Dies kann über eine entsprechende Umschlingungsstrecke des Heizzylinders und damit Größe des Umschlingungswinkels α (vgl. die Figur), den Heizwalzendurchmesser und/oder die Folienbahngeschwindigkeit nach Maßgabe der Foliendicke erfolgen. Zusätzlich kann mit anderen Erwärmungsmethoden, wie Strahlungswärme, z.B. mit Infrarotstrahlern, gearbeitet werden. Aufgrund ihres teilweise schmelzeflüssigen Zustands haftet die Folienbahn stärker auf der Walze, was eine Verschiebung des Ablösepunktes in Drehrichtung der Heizwalze zur Folge hat und eine Vergrößerung des Ablösewinkels β (vgl. die Figur) bedeutet. Um ein Ablösen der Folienbahn von der Heizwalze zu ermöglichen und damit ein Abreißen der Folienbahn zu verhindern, wird zweckmäßigerweise eine Heizwalze mit antihaft-beschichteter Oberfläche verwendet, die eine verringerte Haftung gegenüber der teilweise schmelzeflüssigen Folienbahn aufweist. Beispielsweise wird hierzu eine PTFE (Polytetrafluorethylen)-beschichtete Heizwalze verwendet.

Erfindungsgemäß wird die Folie zwischen wenigstens einer Heizwalze und dem gekühlten Walzenspalt gereckt. In der vorliegenden Erfindung bezeichnet der Begriff "Recken" das gleiche wie "Verstrecken" oder "Strecken". Ebenso bedeutet "Reckverhältnis" das gleiche wie "Streckverhältnis" oder "Verstreckverhältnis". Ein Recken, Strecken oder Verstrecken der Folie bedeutet eine Dehnung der Folie in die angegebene Richtung, was zu einer Verringerung der Foliendicke führt. Die Folie wird erfindungsgemäß in Maschinen- oder Längsrichtung (*md*) gereckt, beispielsweise durch eine unterschiedliche Geschwindigkeit der Heiz- und Kühlwalzen. Beispielsweise bedeutet ein Reckverhältnis von 1:1,5, dass die Foliendicke von z.B. 15 µm auf 10 µm verringert wird. Erfindungsgemäß wesentlich ist, dass sich die Folienbahn während des Reckvorgangs im teilweise geschmolzenen Zustand befindet.

Das Reckverhältnis hängt von der Folienrezeptur und den gewählten Verfahrensparametem ab und beträgt vorzugsweise wenigstens 1:1,2, bevorzugt wenigstens 1: 1,5, insbesondere wenigstens 1:2, stärker bevorzugt wenigstens 1:2,5, mehr bevorzugt wenigstens 1:3 oder wenigstens 1:4.

In einer bevorzugten Ausführungsform der Erfindung wird das Recken dadurch bewirkt, dass die den gekühlten Walzenspalt bildenden Kühlwalzen bei einer höheren Geschwindigkeit als die Heizwalze angetrieben werden. In einer anderen bevorzugten Ausführungsform der Erfindung sind vor dem Kühlwalzenspalt zwei oder mehr Walzen vorhanden, von denen wenigstens zwei mit unterschiedlicher Geschwindigkeit betrieben werden, so dass die Folienbahn zwischen diesen beiden Walzen gereckt wird, und wobei wenigstens die erste der zwei oder mehreren Walzen als Heizwalze ausgebildet ist. Es ist auch möglich, dass die zweite und die gegebenenfalls weiteren Walzen ebenfalls als Heizwalze ausgebildet sind. Insbesondere bei Vorhandensein mehrerer Walzen ist aber auch möglich, dass eine der Walzen als Kühlwalze ausgebildet ist. Eine Kühlwalze bewirkt eine einseitige Abkühlung der Folienbahn und führt damit zu einer langsamen Abkühlung der Folie. Im Gegensatz dazu bewirkt der erfindungsgemäß vorgesehene Kühlwalzenspalt aufgrund der zwei Kühlwalzen eine zweiseitige Kühlung der Folienbahn, was eine schnelle Kühlung herbeiführt. Wenn eine Kühlwalze eingesetzt wird, ist vor dem Kühlwalzenspalt nochmals eine Erwärmung der Folienbahn in den teilweise geschmolzenen Zustand erforderlich, was zweckmäßigerweise wieder über eine Heizwalze erfolgen kann. Möglich sind also beispielsweise die Anordnungen Heizwalze - Heizwalze - gekühlter Walzenspalt oder Heizwalze - Kühlwalze - Heizwalze - gekühlter Walzenspalt.

Es ist auch möglich, die Folienbahn zusätzlich einer Querverstreckung zu unterwerfen. Ein solches biaxiales Verstrecken kann beispielsweise durch auf dem Markt erhältliche Reckmaschinen, z.B. von der Firma Brückner, erreicht werden. Hierbei ist darauf zu achten, dass der erfindungsgemäße teilweise schmelzflüssige Zustand während des Reckvorgangs eingehalten wird.

Bei dem erfindungsgemäßen Verfahren zur Herstellung eines Vlies-Folien-Laminats sind vor dem gekühlten Walzenspalt wenigstens zwei Heizwalzen vorhanden, die mit unterschiedlicher Geschwindigkeit betrieben werden, so dass die Folienbahn zwischen beiden gereckt wird. Zusätzlich läuft eine Vliesbahn auf die zweite der beiden Heizwalzen zu, wobei entweder die Folienbahn oder die Vliesbahn auf der Heizwalze aufliegen kann. Möglich ist auch das Zuführen mehrerer Vliesbahnen. Ein solches Thermolaminier-Verfahren kann beispielsweise wie in der WO 2006/024394 beschrieben durchgeführt werden. Ähnlich wie oben beim Recken der Folie beschrieben, ist auch beim Thermolaminieren eine Kühlwalze zwischen den wenigstens zwei Heizwalzen möglich, insbesondere wenn mehrere Heizwalzen vorhanden sind. Beim Thermolaminieren wird die Ausgangsfolienbahn zusammen mit einer Ausgangsvliesbahn, deren Schmelzpunkt oberhalb des Kristallitschmelzpunktes des Polymermaterials der Ausgangsfolienbahn liegt, auf eine Temperatur über dem Kristallitschmelzpunkt des Polymermaterials der Ausgangsfolienbahn und unter dem Schmelzpunkt der Ausgangsvliesbahn erwärmt. Das Polymermaterial der Ausgangsfolienbahn bezeichnet hierbei die niedrig schmelzende Polymerkomponente und, in Abhängigkeit von der Morphologie und dem chemischen Aufbau der Ausgangsvliesbahn, gegebenenfalls zusätzlich die gesamte hoch schmelzende Polymerkomponente oder einen Teil davon. Der chemische Aufbau des Polymermaterials der Ausgangsfolienbahn sollte dem chemischen Aufbau der Vliesbahn angepasst sein, d.h. die Schmelzpunkte und Rohstoffe sollten aufeinander abgestimmt sein. Ebenso wie in der WO 2006/024394 beschrieben, sollten die zu laminierenden Bahnen zumindest in einer Rezepturkomponente eine ähnliche Morphologie aufweisen, damit eine ausreichende Verbundhaftung erzielt werden kann. Im vorliegenden Fall bedeutet dies, dass die Heizwalze, bei der die Ausgangsvliesbahn zugeführt wird, die Ausgangsfolienbahn derart erwärmt, dass sich die Polymerkomponente, die den Verbund ergibt, im schmelzeflüssigen Zustand befindet. Die Temperatur der Heizwalzen beim Recken und beim Thermolaminieren können sich also in Abhängigkeit von der Zusammensetzung der Ausgangsfolienbahn und der Ausgangsvliesbahn voneinander unterscheiden, insbesondere kann die Heizwalze, bei der die Vliesbahn zugeführt wird, eine höhere Temperatur aufweisen. Dies sei an folgendem Beispiel veranschaulicht. Ist die verwendete Ausgangsvliesbahn auf Basis eines Polypropylens und hat einen Schmelzpunkt im Bereich von 160 bis 165 °C, so sollte sich beim Polymermaterial der Ausgangsfolienbahn ein Polypropylen als verbundgebende Komponente im schmelzeflüssigen Zustand befinden, unabhängig davon ob dieses Polypropylen zur niedrig oder hoch schmelzenden Polymerkomponente der Ausgangsfolienbahn beim Recken gehört. In diesem Fall wäre in der Regel kein ausreichender Verbund gegeben, wenn sich nur ein Polyethylen im schmelzeflüssigen Zustand befindet. Übertragen auf eine Ausgangsfolienrezeptur mit 35 Gew.-% LDPE (Schmelzpunkt 112 °C), 20 Gew.-% LLDPE-Buten (Schmelzpunkt 121 °C), 10 Gew.-% Polypropylen (Schmelzpunkt 162 °C), 30 Gew.-% Random-Polypropylen-Copolymer (Schmelzpunkt 140 °C) und 5 Gew.-% TiO₂-Weiß-Konzentrat, Farbe und Additive, wobei die Bestandteile die gleichen wie die im Beispiel unten in Tabelle I angegebenen Bestandteile sind, würde dies bedeuten, dass sich beim Thermolaminiervorgang das LDPE, das LLDPE-Buten und das Random-Polypropylen-Copolymer (Schmelzpunkt 140 °C) im schmelzeflüssigen Zustand befinden, hingegen nicht das Polypropylen (Schmelzpunkt 162 °C). Das heißt, die Heizwalze, bei der die Vliesbahn zugeführt wird, muss eine entsprechende Erwärmung der Ausgangsfolienbahn, beispielsweise auf 142 °C oder 143 °C, sicherstellen. Bei dieser Temperatur wird ein ausreichender Verbund zum Polypropylen-Vlies erreicht und es besteht nicht die Gefahr eines Anschmelzens des Vlieses. Das Recken findet dagegen bei einer niedrigeren Temperatur statt, beispielsweise bei 124 °C, so dass sich das LDPE und das LLDPE-Buten im schmelzeflüssigen Zustand befinden, nicht aber das Polypropylen (Schmelzpunkt 162 °C) und das Random-Polypropylen-Copolymer (Schmelzpunkt 140 °C).

Die Ausgangsvliesbahn wird in an sich bekannter Weise hergestellt und ist ebenso wie in der WO 2006/024394 bevorzugt auf Basis eines thermoplastischen Polymers, z.B. Fasern aus PE, PP, Polyester (PET), Rayon, Zellulose, Polyamid (PA) oder Mischungen davon. Besonders bevorzugt sind z.B. Vliese aus Spinn- oder Stapelfasern auf Basis von PP, PE oder PET, sowie Vliese aus Mischungen von PP und PE oder Mischungen von PET und PP oder PE. Es ist auch möglich, zwei-oder mehrlagige Vliese einzusetzen.

Erfindungsgemäß wird die Folienbahn nach der Erwärmung durch einen gekühlten Walzenspalt geführt. Bei Herstellung eines Vlies-Folien-Laminats werden die Folienbahn und die Vliesbahn zusammen nach der Erwärmung durch den gekühlten Walzenspalt geführt und miteinander verbunden. Die den Kühlwalzenspalt bildenden Walzen sind so gekühlt, dass eine schnelle und schlagartige Abkühlung erreicht wird. Eine Abkühlung auf eine Temperatur unterhalb des Kristallitschmelzpunktes der niedrig schmelzenden Polymerkomponente, vorzugsweise auf wenigstens 5 °C °C darunter, insbesondere auf wenigstens 10 °C darunter, ist zweckmäßig. Bevorzugte Abkühlbereiche sind 5 bis 10 °C, stärker bevorzugt 10 bis 30 °C, unterhalb des Kristallitschmelzpunktes der niedrig schmelzenden Polymerkomponente. Beispielsweise kann die Kühlung der Walzen mit Wasser in einem Temperaturbereich von 5 bis 20 °C, z.B. Wasser von etwa 10 °C, erfolgen. Der Abstand zwischen der letzten Heizwalze und dem Kühlwalzenspalt sollte hierbei wegen möglicher Wärmeverluste nicht zu groß sein, wobei durch die Abmessungen der Walzen für einen Mindestabstand eine Grenze gesetzt ist. Bei dem Kühlwalzenspalt kann es sich im einfachsten Fall z.B. um einen Glattwalzenspalt mit zwei glatten Walzen handeln. Im Fall von Hygienefolien wird der Walzenspalt jedoch vorzugsweise von einem Walzenpaar mit einer Strukturwalze und einer Glattwalze gebildet, wodurch die Folienbahn eine strukturierte Oberfläche erhält. Bevorzugte Strukturen im Hygienebereich sind Mikrostrukturen, z.B. ein Pyramidenstumpf. Vorzugsweise besteht der gekühlte Walzenspalt aus einer Stahlwalze und einer im Gegendruck arbeitenden Gummiwalze, wobei die Stahlwalze mit der strukturierten Oberfläche versehen ist.

Erfindungsgemäß kann die Geschwindigkeit der den Kühlwalzenspalt bildenden Walzen so gewählt werden, dass diese höher als die Geschwindigkeit der Heizwalze oder, bei Verwendung mehrerer Heizwalzen, der letzten Heizwalze ist, so dass die Folie dazwischen gereckt wird. Alternativ oder zusätzlich kann, wie oben beschrieben, die Folie auch zwischen zwei Walzen vor dem Kühlwalzenspalt gereckt werden. Diese Ausführungsform ist insbesondere dann von Interesse, wenn der Abstand zwischen Heizwalze und Kühlwalzenspalt möglichst gering gehalten werden sollte, um z.B. Einschnürungen beim Reckvorgang zu vermeiden. Der Reckvorgang findet dann zwischen zwei Heizwalzen statt, deren Abstand beliebig verringert werden kann. Von der zweiten Heizwalze wird dann die gereckte Folie ohne weiteres Recken oder mit geringerem weiterem Recken in den Kühlwalzenspalt geführt.

In Abhängigkeit von den Folienparametern und anderen Verfahrensbedingungen bewegen sich die Folienbahngeschwindigkeiten im Bereich von 50 bis 900 m/min. Die Geschwindigkeit der Heizwalze(n) beträgt vorzugsweise 50 bis 600 m/min, insbesondere 100 bis 400 m/min. Die Geschwindigkeit der den Kühlwalzenspalt bildenden Walzen beträgt vorzugsweise 75 bis 900 m/min, insbesondere 150 bis 600 m/min. Die Geschwindigkeiten der Heizwalze(n) und Kühlwalzen werden so gewählt, dass in Abhängigkeit von der Folienrezeptur und den gewählten Verfahrensparametern das gewünschte Reckverhältnis erreicht wird.

Das erfindungsgemäße Verfahren ermöglicht die Herstellung von Folien mit sehr geringer Foliendicke von z. B. 10, 8, 6 oder auch nur 5 µm. Bevorzugte ungefüllte Folien weisen eine Dicke im Bereich von 2 bis 13 µm auf oder haben ein Flächengewicht von 1 bis 15 g/m². Gefüllte Folien weisen bevorzugt die gleichen Werte für Flächengewichte auf.

Die erfindungsgemäß erhaltenen Folien verfügen, trotzdem sie sehr dünn und weich (haptisch ansprechend) sind, über ausgezeichnete mechanische Eigenschaften und über zusätzlich noch immer hohe Durchstoßfestigkeiten (d.h. Widerstand gegenüber Superabsorberkörnern z.B. in Windeln) und hohe Thermostabilitäten (d.h. Widerstand gegenüber Hotmelt-Klebern). Es war überraschend, dass es überhaupt möglich ist, derartig dünne Folien herzustellen.

Erfindungsgemäß erhaltene Folien können in bekannter Weise weiterverarbeitet werden, wobei die Herstellung von Vlies-Folien-Laminaten besonders bevorzugt ist. Zur Herstellung solcher Laminate können sie mit Klebstoffen, bevorzugt inline, verklebt werden. Daneben können Vlies-Folien-Laminate auch durch dem Fachmann bekanntes Thermobonding hergestellt werden, bei dem das Material von einer erfindungsgemäß erhaltenen Folie und/oder von Vlies punktuell mittels zweier beheizter Walzen, meist einer Prägewalze (gravierte Stahlwalze) und einer glatten Stahlwalze als Gegenwalze, durch hohe Temperatur und Druck angeschmolzen wird und dadurch die Folie und das Vlies miteinander verbunden werden. Darüber hinaus können Vlies-Folien-Laminate wie oben beschrieben auch mittels Thermolaminierung hergestellt werden. Eine Thermolaminierung ist insbesondere bei sehr dünnen Folien, z.B. 4 g/m² bevorzugt. Die hergestellten Vlies-Folien-Laminate können in an sich bekannter Weise weiterverarbeitet werden, wobei auch ein Recken in Maschinen- oder Querrichtung oder in beide Richtungen möglich ist.

Die Figur zeigt eine bevorzugte Ausführungsform zur Durchführung des erfindungsgemäßen Verfahrens, bei dem das Recken durch eine höhere Geschwindigkeit der den gekühlten Walzenspalt bildenden Walzen im Vergleich zur Heizwalze erzielt wird. Von einer Rolle 1 läuft eine Ausgangsfolienbahn 2 über Umlenkwalzen 3 und 4 und eine Anpresswalze 5 auf eine Heizwalze 6. Bei der Heizwalze 6 handelt es sich z.B. um eine antihaftend beschichtete Stahlwalze, die mittels Wärmezufuhr auf die gewünschte Oberflächentemperatur erhitzt wird. Die Folienbahn läuft dann auf der Heizwalze 6 und wird dabei erfindungsgemäß erwärmt. Bei dem Umschlingungswinkel α handelt es sich um denjenigen Winkel, der gebildet wird vom ersten Berührungspunkt der Ausgangsfolienbahn 2 mit der Heizwalze 6 bis zu demjenigen Punkt in Drehrichtung der Heizwalze 6 gesehen, an dem die Ablösung der Folienbahn von der Heizwalze erfolgt. Von der Heizwalze 6 läuft die Folienbahn am Ablösepunkt A unter einem Ablösewinkel β in einen Kühlwalzenspalt, der von den Walzen 7 und 8 gebildet wird. Die Walze 8 ist vorzugsweise als Strukturwalze ausgebildet, wodurch die Folienbahn eine strukturierte Oberfläche erhält. Das Walzenpaar 7/8 ist vorzugsweise wassergekühlt, z.B. mit Wasser von etwa 10 °C. Die den Kühlspalt bildenden Walzen 7 und 8 werden so angetrieben, dass gegenüber der Bahngeschwindigkeit der Heizwalze 6 eine höhere Geschwindigkeit besteht, so dass der gewünschte Reckgrad erreicht wird. Dabei verursacht ein Reckgrad zwischen Heizwalze und Walzenspalt eine Verringerung des Ablösewinkels β. Nach dem Walzenpaar 7/8 wird die Folie abgenommen.

Die Erfindung ermöglicht aufgrund der Herstellung von Folien äußerst geringer Dicken eine Einsparung an Rohstoffen und trägt somit zur Ressourcenschonung und Nachhaltigkeit bei. Sie leistet somit einen Beitrag zur Umweltschonung. Dies gilt insbesondere für Folien auf dem Hygienesektor, in dem die Folien in starkem Maße als Bestandteile von Wegwerfprodukten verwendet werden.

Die erfindungsgemäß erhaltenen Folien und Vlies-Folien-Laminate finden Verwendung im Hygiene- oder Medikalbereich, z.B. als Wäscheschutzfolie oder allgemein als flüssigkeitsundurchlässige Sperrschicht, insbesondere als Backsheets in Windeln, Damenbinden, Betteinlagen oder in ähnlichen Produkten.

Die Erfindung wird anhand des nachfolgenden Beispiels näher erläutert.

### BEISPIEL

Eine Ausgangsfolienbahn wurde nach dem Blasverfahren mit einer Rezeptur gemäß Tabelle I hergestellt, wobei die Folie mit dem Polypropylen mit dem niedrigeren Schmelzpunkt (140 °C) als Außenschicht coextrudiert wurde.

**TABELLE I**

| Folienrezeptur | | |
|---|---|---|
| Menge in Gewichtsteile | Bestandteil | Kristallitschmelzpunkt, °C |
| 55 | LDPE | 112 |
| 20 | LLDPE-Buten¹ | 121 |
| 10 | Polypropylen | 162 |
| 10 | Random-Polypropylen-Copolymer² | 140 |
| 5 | TiO₂ -Weiß-Konzentrat, Farbe und Additive | - |

| | | |
|---|---|---|
| ¹ MFR 1,0 bei 190°C/2,16 kg ² Propylen-Ethylen-Copolymer mit 10 Gew.-% Ethylen | | |

Die Bedingungen beim Blasen des Folienschlauches sind aus nachstehender Tabelle II ersichtlich.

**TABELLE II**

| Blasbedingungen | |
|---|---|
| Ringdüse | 550 mm Durchmesser |
| Düsenspalt | 1,2 mm |
| Schlauchdurchmesser | 1590 mm |
| Folienflächengewicht | 14 g/m² |
| Extrudertemperatur | 240 °C |

Der erhaltene Folienschlauch wurde in Längsrichtung aufgeschnitten und auf zwei Rollen aufgewickelt. Die Folienbreite betrug 2,5 m.

Diese Ausgangsfolienbahn wurde dem in der Figur gezeigten Verfahren wie folgt unterworfen. Nach dem Abziehen der Ausgangsfolienbahn 2 von der Rolle 1 läuft diese über die Umlenkwalzen 3, 4 und die Anpresswalze 5 auf die Heizwalze 6. Bei der Heizwalze (HZ) 6 handelt es sich um eine antihaftend beschichtete Stahlwalze, die mittels Wärmezufuhr auf eine Oberflächentemperatur gemäß Tabelle III erhitzt wird. Die Heizwalze 6 wird mit einer Bahngeschwindigkeit von 100 m/min angetrieben. Von der Heizwalze 6 läuft die Folienbahn in den von dem Walzenpaar 7/8 gebildeten Kühlwalzenspalt, der mit einer höheren Bahngeschwindigkeit als die Heizwalze gemäß gewünschtem Reckgrad betrieben wird. Aus der Differenzgeschwindigkeit von Heizwalze zu Kühlwalzenspalt ergibt sich der Reckgrad. Beispielsweise ergeben eine Heizwalzengeschwindigkeit von 100 m/min und eine Kühlwalzengeschwindigkeit von 300 m/min einen Reckgrad von 100:300 oder 1:3. Die Walze 8 ist als Glattwalze oder als Walze mit strukturierter Oberfläche ausgebildet. Das Walzenpaar 7/8 ist wassergekühlt (etwa 15°C). Die den Spalt bildenden Walzen 7/8 werden so angetrieben, dass die unten in Tabelle III angegebenen Reckgrade erzielt werden. Dadurch konnten Folien mit dem in Tabelle III angegebenen Flächengewicht erhalten werden. Die Tabelle III zeigt auch Versuche, bei denen nicht ausreichend erwärmt wurde, was zu Abrissen der Folien führte.

**TABELLE III**

| HZ-Temp. | Reckgrad | Produktion von > 5min | Flächengewicht [g/m²] |
|---|---|---|---|
| 105°C | 1:1,50 | Abriss | - |
| 117°C | 1:2,00 | möglich | 7,0 |
| 117°C | 1:3,50 | Abriss | - |
| 124°C | 1:3,50 | möglich | 4,0 |

Überraschenderweise wurde gefunden, dass Reckgrade von mehr als 1:1,50 möglich sind, sobald sich die Ausgangsfolienbahn im schmelzeflüssigen Zustand der niedrigst schmelzenden Polyethylen-Komponente (LDPE) während des Reckvorgangs befindet. So konnte bei einer Recktemperatur (Heizzylinder-oberflächentemperatur) von 117°C (55% LDPE-Anteil im schmelzeflüssigen Zustand) ein Reckgrad von 1:2,0 erreicht werden, d.h. eine 14 g/m² Ausgangsfolie konnte auf 7,0 g/m² gereckt werden. Bei einer Recktemperatur von 124°C (55% LDPE- und 20% LLDPE-Anteil im schmelzeflüssigen Zustand) konnte sogar ein Reckgrad von 1:3,5 erreicht werden, d.h. eine 14 g/m² Ausgangsfolie konnte auf 4,0 g/m² gereckt werden.

Das Beispiel zeigt, dass das erfindungsgemäße Verfahren die Herstellung von Folien mit sehr geringem Flächengewicht ermöglicht.

Die erhaltenen dünnen Folien können anschließend zur besseren Handhabung mit Vliesen zu Laminaten verbunden werden. Geeignete Verfahren hierfür sind Kleben. Alternativ kann ein Thermolaminieren wie oben beschrieben durchgeführt werden, wobei auf die Polypropylen-Außenschicht das Vlies laminiert wird.

## Patentansprüche

1. Verfahren zur Herstellung eines Vlies-Folien-Laminats, wobei das Verfahren folgende Schritte umfasst:
Erwärmen einer Ausgangsfolienbahn aus thermoplastischem Polymermaterial, das wenigstens eine niedrig schmelzende Polymerkomponente und wenigstens eine hoch schmelzende Polymerkomponente enthält, bis zum teilweise geschmolzenen Zustand, bei dem sich die wenigstens eine niedrig schmelzende Polymerkomponente im schmelzeflüssigen Zustand und die wenigstens eine hoch schmelzende Polymerkomponente nicht im schmelzeflüssigen Zustand befindet, durch wenigstens eine Heizwalze, und
Abkühlen durch Führen der teilweise geschmolzenen Folienbahn durch einen gekühlten Walzenspalt,
wobei die Folie zwischen der wenigstens einen Heizwalze und dem gekühlten Walzenspalt gereckt wird, und
wobei vor dem gekühlten Walzenspalt wenigstens zwei Heizwalzen vorhanden sind, die mit unterschiedlicher Geschwindigkeit betrieben werden, so dass die Folienbahn zwischen der ersten und zweiten Heizwalze gereckt wird, und wobei zusätzlich eine Vliesbahn über die zweite Heizwalze mitgeführt und zusammen mit der Folienbahn durch den gekühlten Walzenspalt geführt wird.

2. Verfahren nach Anspruch 1, **dadurch gekennzeichnet, dass** eine Ausgangsfolienbahn mit 15 bis 85 Gew.-% niedrig schmelzender Polymerkomponente und 85 bis 15 Gew.-% hoch schmelzender Polymerkomponente, bezogen auf 100 Gew.-% niedrig und hoch schmelzende Polymerkomponente, verwendet wird.

3. Verfahren nach Anspruch 1 oder 2, **dadurch gekennzeichnet, dass** eine Ausgangsfolienbahn mit wenigstens einem Polyethylen als niedrig schmelzender Polymerkomponente und mit wenigstens einem Polypropylen als hoch schmelzender Polymerkomponente verwendet wird.

4. Verfahren nach einem der vorhergehenden Ansprüche 1 bis 3, **dadurch gekennzeichnet, dass** die Erwärmung der Ausgangsfolienbahn auf 5 bis 20 °C unterhalb des Kristallitschmelzpunktes der wenigstens einen hoch schmelzenden Polymerkomponente erfolgt.

5. Verfahren nach einem der vorhergehenden Ansprüche, **dadurch gekennzeichnet, dass** die Folienbahn bei einem Reckverhältnis von wenigstens 1:1,2, bevorzugt wenigstens 1:1,5, insbesondere wenigstens 1:2, gereckt wird.

6. Verfahren nach einem der vorhergehenden Ansprüche, **dadurch gekennzeichnet, dass** die den gekühlten Walzenspalt bildenden Walzen bei einer höheren Geschwindigkeit als die wenigstens eine Heizwalze angetrieben werden.

7. Verfahren nach einem der vorhergehenden Ansprüche, **dadurch gekennzeichnet, dass** vor dem gekühlten Walzenspalt zwei Walzen vorhanden sind, die mit unterschiedlicher Geschwindigkeit betrieben werden, so dass die Folienbahn zwischen der ersten und zweiten Walze gereckt wird, und wobei wenigstens die erste der zwei Walzen als Heizwalze ausgebildet ist.

8. Verfahren nach einem der vorhergehenden Ansprüche, **dadurch gekennzeichnet, dass** die Folienbahn im gekühlten Walzenspalt einer Abkühlung auf wenigstens 10 bis 30 °C unterhalb des Kristallitschmelzpunktes der wenigstens einen niedrig schmelzenden Polymerkomponente unterworfen wird.

9. Vlies-Folien-Laminat, erhältlich durch ein Verfahren nach einem der Ansprüche 1 bis 8, wobei die Folienbahn eine Dicke im Bereich von 2 bis 13 µm aufweist.

10. Verwendung des Vlies-Folien-Laminats nach Anspruch 9 im Hygiene- oder Medikalbereich, insbesondere für Backsheets in Windeln, für Betteinlagen oder Damenbinden.

## Claims

1. A process for the manufacture of a non-woven fabric-film laminate, wherein the process comprises the following steps:
heating of a starting film web of a thermoplastic polymer material containing at least one low-melting polymer component and at least one high-melting polymer component, to an at least partly molten state in which the at least one low-melting polymer component exists in a molten liquid state and the at least one high-melting polymer component does not exist in the molten liquid state, by at least one heating roller and
cooling down by passing the partly molten film web through a cooled roller nip,
the film being stretched between the at least one heating roller and the cooled roller nip, and
wherein upstream of the cooled roller nip at least two heating rollers are present, which are operated at different velocities, so that the film web is stretched between the first and the second heating rollers, and wherein, additionally, a non-woven fabric web is passed over the second heating roller and is passed through the cooled roller nip together with the film web.

2. The process according to claim 1, **characterized in that** a starting film web with 15 to 85 % by weight of low-melting polymer component and 85 to 15 % by weight of high-melting polymer component, based on 100 % by weight of low-melting and high-melting polymer components, is used.

3. The process according to claim 1 or 2, **characterized in that** a starting film web with at least one polyethylene serving as a low-melting polymer component and at least one polypropylene serving as a high-melting polymer component is used.

4. The process according to any one of the preceding claims 1 to 3, **characterized in that** the heating of the starting film web is performed up to 5 to 20 °C below the crystallite melting point of the at least one high-melting polymer component.

5. The process according to any one of the preceding claims, **characterized in that** the film web is stretched at a stretching ratio of at least 1:1.2, preferably at least 1:1.5, in particular at least 1:2.

6. The process according to any one of the preceding claims, **characterized in that** the rollers forming the cooled roller nip are driven at a higher velocity than the at least one heating roller.

7. The process according to any one of the preceding claims, **characterized in that** upstream of the cooled roller nip two rollers are present, which are driven at different velocities, such that the film web is stretched between the first and the second rollers and wherein at least the first of the two rollers is designed as a heating roller.

8. The process according to any one of the preceding claims, **characterized in that** the film web is subjected to cooling in the cooled roller nip to at least 10 to 30°C below the crystallite melting point of the at least one low-melting polymer component.

9. A non-woven fabric-film laminate obtainable by a process according to any of claims 1 to 8, wherein the film web has a thickness in the range of 2 to 13 µm.

10. Use of the non-woven fabric-film laminate according to claim 9 in the hygiene or medical field, in particular for back sheets of diapers, for mattress protectors or sanitary napkins.

## Revendications

1. Procédé de fabrication d'un stratifié feuille/non-tissé, lequel procédé comprend les étapes suivantes :
chauffage d'une bande de feuille de départ en matériau polymère thermoplastique, qui comprend au moins un composant polymère à bas point de fusion et au moins un composant polymère à haut point de fusion, jusqu'à l'état partiellement fondu, dans lequel ledit au moins un composant polymère à bas point de fusion se trouve à l'état fondu liquide et ledit au moins un composant polymère à haut point de fusion ne se trouve pas à l'état fondu liquide, par au moins un rouleau chauffant, et
refroidissement par guidage de la bande de feuille partiellement fondue à travers une fente de laminage refroidie,
la feuille étant étirée entre ledit au moins un rouleau chauffant et la fente de laminage refroidie, et
au moins deux rouleaux chauffants étant présents avant la fente de laminage refroidie, lesquels tournent à une vitesse différente, de sorte que la bande de feuille est étirée entre le premier et le deuxième rouleau chauffant, et une bande de non-tissé étant entraînée en plus par le deuxième rouleau chauffant et guidée avec la bande de feuille à travers la fente de laminage refroidie.

2. Procédé selon la revendication 1, **caractérisé en ce que** l'on utilise une bande de feuille de départ avec 15 à 85 % en poids de composant polymère à bas point de fusion et 85 à 15 % en poids de composant polymère à haut point de fusion, par rapport à 100 % en poids de composant polymère à bas et haut point de fusion.

3. Procédé selon la revendication 1 ou 2, **caractérisé en ce que** l'on utilise une bande de feuille de départ avec au moins un polyéthylène comme composant polymère à bas point de fusion et avec au moins un polypropylène comme composant polymère à haut point de fusion.

4. Procédé selon l'une des revendications précédentes 1 à 3, **caractérisé en ce que** la bande de feuille de départ est chauffée à entre 5 et 20 °C au-dessous de la température de fusion des cristallites dudit au moins un composant polymère à haut point de fusion.

5. Procédé selon l'une des revendications précédentes, **caractérisé en ce que** la bande de feuille est étirée selon un rapport d'étirage d'au moins 1:1,2, de préférence d'au moins 1:1,5, en particulier d'au moins 1:2.

6. Procédé selon l'une des revendications précédentes, **caractérisé en ce que** les rouleaux formant la fente de laminage refroidie sont entraînés à une vitesse plus élevée que ledit au moins un rouleau chauffant.

7. Procédé selon l'une des revendications précédentes, **caractérisé en ce que** deux rouleaux sont présents avant la fente de laminage refroidie, lesquels tournent à une vitesse différente, de sorte que la bande de feuille est étirée entre le premier et le deuxième rouleau, au moins le premier des deux rouleaux étant conçu comme rouleau chauffant.

8. Procédé selon l'une des revendications précédentes, **caractérisé en ce que**, dans la fente de laminage refroidie, la bande de feuille est soumise à un refroidissement à au moins 10 à 30 °C au-dessous de la température de fusion des cristallites dudit au moins un composant polymère à bas point de fusion.

9. Stratifié feuille/non-tissé pouvant être obtenu par un procédé selon l'une des revendications 1 à 8, dans lequel la bande de feuille présente une épaisseur comprise entre 2 et 13 µm.

10. Utilisation du stratifié feuille/non-tissé selon la revendication 9 dans le domaine hygiénique ou médical, en particulier pour des backsheets dans des couches-culottes, pour des alèses ou des serviettes hygiéniques.
